# EUROPEAN PATENT APPLICATION

(11) **EP 3 153 842 A1**
(43) Date of publication of application: **12.04.2017**
(21) Application number: 15807547.3
(22) Date of filing: 09.06.2015
(51) Int. Cl.: G01N 17/00

(54) **METHOD FOR EVALUATING WEATHERABILITY OF FLUORINATED COATING FILM**

(30) Priority: 09.06.2014 JP 2014118759; 01.09.2014 JP 2014177540; 01.09.2014 JP 2014177541
(71) Applicant: Asahi Glass Company, Limited, Tokyo 100-8405 (JP)
(72) Inventor: SAITO, Shun, Tokyo 100-8405 (JP); AIKAWA, Masataka, Tokyo 100-8405 (JP)
(74) Representative: Müller-Boré & Partner Patentanwälte PartG mbB
(86) International application number: PCT/JP2015/066569
(87) International publication number: WO 2015/190463

(57) **Abstract**

To provide a method for evaluating the weather resistance by which the weather resistance of a fluorinated coating film can be evaluated accurately in a short time.

A method for evaluating the weather resistance of a fluorinated coating film, which comprises a fluorinated coating film deterioration step (I) comprising a step (ia) of exposing the fluorinated coating film to xenon arc radiation and a step (ib) of attaching an aqueous hydrogen peroxide solution to the fluorinated coating film under xenon arc radiation to wet the fluorinated coating film, and an evaluation step (II) of comparing characteristics of the fluorinated coating film as between before and after the deterioration step (I) to evaluate the weather resistance of the fluorinated coating film.

## Description

### TECHNICAL FIELD

The present invention relates to a method for evaluating the weather resistance of a fluorinated coating film.

### BACKGROUND ART

As a method for evaluating the weather resistance of various substances, etc., a method of exposing a substance (test piece) to natural environment and confirming changes of various properties with time (outdoor exposure test) has been known. However, by such a method, evaluation of the weather resistance will take long since the test piece slowly deteriorates.

Accordingly, as a method for evaluating the weather resistance in a short time, an accelerated weathering test has been known. For example, as a method for evaluating the weather resistance of a coating film, "Accelerated weathering and exposure to artificial radiation (Exposure to filtered xenon-arc radiation)" as defined in JIS K5600-7-7 (2008) has been known. In this method, a test piece is exposed to xenon arc radiation while the test piece is wetted with water to accelerate deterioration of the coating film.

Further, Patent Document 1 discloses as a method for evaluating a coating film having titanium oxide dispersed in a resin matrix, a method comprising a first deterioration accelerating step of applying oxygen plasma and a second deterioration accelerating step of applying e.g. an aqueous hydrogen peroxide solution to the surface of the test piece.

Patent Document 2 discloses a method for evaluating the weather resistance comprising irradiating a coating plate coated with a coating material containing an alkyd resin, a melamine resin and titanium oxide together with an aqueous hydrogen peroxide solution, and discloses that the results obtained by this evaluation method and results of outdoor exposure are favorably reproducible.

### PRIOR ART DOCUMENTS

### PATENT DOCUMENTS

Patent Document 1: JP-A-2001-208675
Patent Document 2: JP-A-2001-262071

### DISCLOSURE OF INVENTION

### TECHNICAL PROBLEM

However, according to studies by the present inventors, when the weather resistance of a fluorinated coating film containing a fluororesin was evaluated by the method as defined in JIS K5600-7-7 (2008), no valid evaluation results tended to be obtained.

That is, usually, a fluorinated coating film is excellent in the weather resistance as compared with other resin coating films. Accordingly, even when a plurality of test pieces having different types of fluorinated coating film are evaluated by the method as defined in JIS K5600-7-7 (2008), no difference in the weather resistance among the test pieces tended to be confirmed, and the weather resistance of the fluorinated coating film could not appropriately be evaluated.

Further, although Patent Document 1 discloses that the method disclosed therein is effective as a method for evaluating the weather resistance with respect to a resin coating film containing titanium oxide particles, it failed to disclose validity when a fluorinated coating film is evaluated. Further, Patent Document 2 also failed to disclose evaluation of a fluorinated coating film.

The object of the present invention is to provide a method for evaluating the weather resistance of a fluorinated coating film accurately in a short time, and a method for producing a fluorinated coating film comprising the method.

### SOLUTION TO PROBLEM

The present invention provides the following.
[1] A method for evaluating the weather resistance of a fluorinated coating film, which comprises a fluorinated coating film deterioration step (I) comprising a step (ia) of exposing the fluorinated coating film to xenon arc radiation and a step (ib) of attaching an aqueous hydrogen peroxide solution to the fluorinated coating film under xenon arc radiation to wet the fluorinated coating film, and an evaluation step (II) of comparing characteristics of the fluorinated coating film as between before and after the deterioration step (I) to evaluate the weather resistance of the fluorinated coating film.
[2] The method for evaluating the weather resistance according to [1], wherein the irradiance of the xenon arc radiation in the step (ia) is from 50 to 400 W/m² within a wavelength range of from 300 to 400 nm.
[3] The method for evaluating the weather resistance according to [1] or [2], wherein in the step (ib), the concentration of the aqueous hydrogen peroxide solution is from 0.05 to 10.0 mass%.
[4] The method for evaluating the weather resistance according to any one of [1] to [3], wherein the step (ib) is carried out more than once.
[5] The method for evaluating the weather resistance according to any one of [1] to [4], which further comprises a drying step (ic) after the step (ib) is carried out and before the evaluation step (II) is carried out.
[6] The method for evaluating the weather resistance according to any one of [1] to [5], wherein the weather resistance is evaluated by at least one selected from the group consisting of glossiness, color difference, outer appearance of the coating film by observation with a scanning electron microscope, mapping of F atoms and titanium atoms by energy dispersive X-ray spectroscopy, and peak variation of fluororesin by infrared analysis.
[7] The method for evaluating the weather resistance according to any one of [1] to [6], wherein the total time of the xenon arc radiation is from 1 to 10,000 hours.
[8] The method for evaluating the weather resistance according to any one of [1] to [7], wherein the fluorinated coating film contains at least one fluororesin in an amount of at least 5 mass% in the fluorinated coating film (100 mass%), and has a film thickness of from 10 to 100 µm.
[9] The method for evaluating the weather resistance according to any one of [1] to [8], wherein the fluorinated coating film is formed by using a powder coating material, a solvent-based coating material or an aqueous coating material.
[10] A method for producing a fluorinated coating film, which comprises forming a fluorinated coating film on a substrate, evaluating the weather resistance of the fluorinated coating film by the method for evaluating the weather resistance as defined in any one of [1] to [9], and selecting a fluorinated coating film which has achieved an optional standard of weather resistance by the evaluation.

### ADVANTAGEOUS EFFECTS OF INVENTION

According to the method for evaluating the weather resistance of a fluorinated coating film of the present invention, the weather resistance of a fluorinated coating film can be evaluated accurately in a short time. Further, according to the method for producing a fluorinated coating film of the present invention, which employs the method for evaluating the weather resistance of the present invention, a fluorinated coating film excellent in the weather resistance can be selected in a short time.

### DESCRIPTION OF EMBODIMENTS

### <Method for evaluating weather resistance>

The method for evaluating the weather resistance of the present invention is a method for evaluating the weather resistance of a fluorinated coating film containing a fluororesin, and comprises a deterioration step (I) and an evaluation step (II).

The deterioration step (I) is a step of accelerating the deterioration of the fluorinated coating film and comprises at least a step (ia) of exposing the fluorinated coating film to xenon arc radiation and a step (ib) of attaching an aqueous hydrogen peroxide solution to the fluorinated coating film under xenon arc radiation to wet the fluorinated coating film.

The evaluation step (II) is a step of comparing characteristics of the fluorinated coating film as between before and after the deterioration step (I) to evaluate the weather resistance of the fluorinated coating film.

By such a method for evaluating the weather resistance, in the deterioration step (I), an aqueous hydrogen peroxide solution is used to wet a test piece, and accordingly oxidative deterioration of the fluorinated coating film is likely to proceed. Accordingly, the weather resistance can be evaluated accurately in a short time even with respect to a fluorinated coating film which is superior in the weather resistance to a usual coating film and of which oxidative deterioration does not proceed so much by e.g. a method disclosed in JIS K5600-7-7 (2008) which employs water to wet the test piece.

The deterioration step (I) preferably comprises a step (ib) of attaching an aqueous hydrogen peroxide solution to the fluorinated coating film under xenon arc radiation to wet it more than once, more preferably comprises a step (ib) of attaching an aqueous hydrogen peroxide solution to the fluorinated coating film under xenon arc radiation to wet it more than once and a step of (ic) of drying the attached aqueous hydrogen peroxide solution after each step (ib). That is, it is preferred that attachment and drying are carried out more than once during xenon arc radiation, whereby the weather in a hot and humid climate zone can be well simulated. The step of attaching an aqueous hydrogen peroxide solution to wet the fluorinated coating film is carried out preferably by spraying an aqueous hydrogen peroxide solution.

The spraying may be carried out in accordance with "Accelerated weathering and exposure to artificial radiation (Exposure to filtered xenon arc radiation)" as defined in JIS K5600-7-7 (2008). Particularly, spraying is carried out preferably by using e.g. a xenon weather meter under an aqueous hydrogen peroxide solution discharge pressure of 0.2 MPa·s with a discharge pore of a discharge nozzle of from 0.3 to 1.0 mm at an aqueous hydrogen peroxide solution discharge flow rate of from 80 to 150 g/m².

Further, after the step (ic) is carried out and before the next step (ib) is carried out, a step (id) of washing the fluorinated coating film with water such as deionized water may be carried out. The step (id) is not necessarily carried out after all the steps (ic), and may be carried out after at least one step (ic). For example, it is preferred to carry out the step (id) at least after the final step (ic) in the deterioration step (I) and before the evaluation step (II). During the step (id), xenon arc radiation is preferably suspended.

The characteristics to be compared of the fluorinated coating film as between before and after the deterioration step (I) in the evaluation step (II) are not particularly limited, and for example, comparison of the glossiness, comparison of the color difference, comparison of the outer appearance of the coating film by observation with a scanning electron microscope, comparison of mapping of F atoms and titanium atoms by energy dispersive X-ray spectroscopy, and peak variation (comparison of e.g. the position or the intensity of the peak) of fluororesin by infrared analysis may be mentioned.

In a case where the glossiness is employed among the characteristics, for example, the proportion of the 60° glossiness of the fluorinated coating film after the deterioration step (I) based on the 60° glossiness of the fluorinated coating film before the deterioration step (I) being 100% is obtained as the glossiness retention (unit: %), based on which the weather resistance can be evaluated. The higher the glossiness retention, the more excellent the weather resistance of the fluorinated coating film.

The glossiness retention may be measured and calculated in accordance with JIS K5600-4-7: 1999 (ISO2813: 1994).

In a case where the color difference is employed among characteristics, for example, colorimetry before the deterioration step (I) and colorimetry after the deterioration step (I) are measured by a colorimeter in accordance with JIS K5600-4-5: 1999. And, the color difference (ΔE) as between before and after the deterioration step (I) is calculated in accordance with JIS K5600-4-6: 1999. The smaller the color difference (ΔE), the more excellent the weather resistance of the fluorinated coating film.

In the deterioration step (I), conditions such as the time for xenon arc radiation, the time for each of spraying (attachment) and drying, the shape of a test chamber, the concentration of the aqueous hydrogen peroxide solution, the relative humidity and temperature of the environment (test chamber) in which the test piece is held, and the irradiance of the light source may be properly set.

The deterioration step (I) is preferably carried out under the following conditions (1) to (5) in accordance with method 1 of "Accelerated weathering and exposure to artificial radiation (Exposure to filtered xenon arc radiation)" as defined in JIS K5600-7-7 (2008). In this specification, the irradiance of the xenon arc radiation is the irradiance within a wavelength region of from 300 to 400 nm and will be referred to simply as (300 to 400 nm).
(1) Spray liquid: A 0.05 to 10.0 mass% aqueous hydrogen peroxide solution.
(2) Relative humidity: 30 to 90%RH.
(3) Black panel temperature: 20 to 70°C.
(4) Irradiance of xenon arc radiation: 50 to 400 W/m² (300 to 400 nm).
(5) Cycle of spraying and drying of spray liquid: spray for 1 to 60 minutes and drying for 1 to 360 minutes.

The concentration of the aqueous hydrogen peroxide solution as (1) spray liquid is preferably from 0.05 to 10.0 mass%, more preferably from 0.07 to 8.0 mass%, particularly preferably from 0.1 to 5.0 mass% with a view to more accurately evaluating the weather resistance, with a view to improving the precision in correlation with natural exposure test, in view of easiness of handling of hydrogen peroxide, etc.

The above (2) relative humidity is preferably from 30 to 90%RH, more preferably from 35 to 85%RH, particularly preferably from 40 to 80%RH, with a view to more accurately evaluating the weather resistance, etc. The relative humidity within the above range is preferred also in that the correlation with natural exposure test is satisfied with respect to the amount of photoradicals generated from titanium oxide when the fluorinated coating film contains titanium oxide as a pigment.

The above (3) black panel temperature is preferably from 20 to 70°C, more preferably from 23 to 67°C, particularly preferably from 25 to 65°C with a view to more accurately evaluating the weather resistance, with a view to reproducing the temperature history of the coating film in natural exposure test, etc.

The above (4) irradiance of xenon arc radiation is preferably from 50 to 400 W/m² (300 to 400 nm), more preferably from 52 to 390 W/m², particularly preferably from 55 to 380 W/m², with a view to more accurately evaluating the weather resistance, with a view to reproducing the wavelength distribution of sunlight, etc. Particularly, the irradiance of xenon arc radiation is still more preferably from 60 to 300 W/m², most preferably from 60 to 200 W/m², with a view to shortening the evaluation time.

The above (5) cycle of spraying and drying of spray liquid is preferably spraying time for 1 to 60 minutes and drying time for 1 to 360 minutes, more preferably spraying time for 2 to 55 minutes and drying time for 2 to 330 minutes, particularly preferably spraying time for 3 to 50 minutes and drying time for 2 to 300 minutes, with a view to more accurately evaluating the weather resistance, with a view to reproducing rainfall in natural exposure test, etc.

An example of the procedure of the above-described deterioration step (I) and evaluation step (II) is as follows. A test piece wetting cycle to be employed in this method may be cycle A or cycle B, and cycle A is employed in the following example. Procedure:
(1 a) A test piece having a fluorinated coating film is prepared.
(2a) The test piece is fixed in a chamber of a xenon weather meter test apparatus.
(3a) The relative humidity in the chamber is adjusted to 70%RH, the black panel temperature is adjusted to 50°C, the irradiance of xenon arc radiation is adjusted to 80 W/m² (300 to 400 nm) and the concentration of the aqueous hydrogen peroxide solution to be sprayed on the test piece is adjusted to 1 mass%.
(4a) The xenon weather meter is operated continuously for 2 hours under the above conditions.
(5a) Spraying of the aqueous hydrogen peroxide solution during driving is conducted in such a manner that spraying is carried out for 3 minutes and suspended for 2 minutes, and this operation is repeated 24 times (that is, the cycle of spraying and drying of spray liquid is spraying for 3 minutes and drying for 2 minutes). During drying, the temperature of the surface of the coating film of the test piece is monitored by a platinum resistant bulb thermometer to be 30 ± 1.0°C.
(6a) Then, the operation under the above conditions is terminated, and the surface of the fluorinated coating film of the test piece is washed by spraying with deionized water for 30 minutes.
(7a) (4a) to (6a) is repeated four times (10 hours in total. 8 hours in total for xenon arc radiation).
(8a) Then, the test piece is discharged, water droplets are removed, and the color difference (for example, using a colorimeter (manufactured by NIPPON DENSHOKU INDUSTRIES CO., LTD., SA4000)) and the glossiness (for example, using a glossimeter (manufactured by NIPPON DENSHOKU INDUSTRIES CO., LTD., PG-1 M)) are measured.
(9a) (7a) and (8a) are repeated for 5 cycles (50 hours in total (excluding the time required for (8a))).
(10a) After completion of (9a), as the case requires, with respect to the surface and the cross section of the fluorinated coating film, a difference with the test piece before the test is confirmed e.g. by a scanning electron microscope, energy dispersive X-ray spectroscopy or infrared analysis.

The time for xenon arc radiation is not particularly limited, but the total radiation time is preferably from 1 to 10,000 hours, more preferably from 3 to 5,000 hours, particularly preferably from 5 to 1,000 hours, with a view to more accurately evaluating the weather resistance of the fluorinated coating film. In the above procedure, the total time for xenon arc radiation is 40 hours.

### <Fluorinated coating film>

The fluorinated coating film to be evaluated in the present invention should contain at least one fluororesin, and may contain at least one component other than the fluororesin.

The thickness of the fluorinated coating film is not particularly limited and is preferably from 10 to 100 µm, more preferably from 15 to 80 µm.

The fluorinated coating film may be laminated on a substrate made of a metal such as aluminum, iron or magnesium or on a coating film other than the fluorinated coating film, and its shape is not limited.

The fluororesin is a polymer compound having fluorine atoms in its molecule and may be a homopolymer or copolymer of a fluoroolefin. In the case of a copolymer, it may, for example, be a copolymer of at least two types of fluoroolefin, a copolymer of at least one type of fluoroolefin and at least one type of a fluorinated monomer other than fluoroolefin, a copolymer of at least one type of fluoroolefin and at least one type of monomer having no fluorine atom, or a copolymer of at least one type of fluoroolefin, at least one type of fluorinated monomer other than fluoroolefin, and at least one type of monomer having no fluorine atoms.

The fluoroolefin is a compound having at least one of hydrogen atoms in a hydrocarbon-type olefin (general formula: (CₙH₂ₙ) substituted with a fluorine atom. In the fluoroolefin, at least one of hydrogen atoms not substituted with a fluorine atom may be substituted with a fluorine atom.

Further, a perfluorinated monomer means a monomer having all the hydrogen atoms bonded to carbon atoms substituted with fluorine atoms.

The fluoroolefin may, for example, be tetrafluoroethylene, chlorotrifluoroethylene, hexafluoropropylene, vinylidene fluoride or vinyl fluoride.

The fluorinated monomer other than fluoroolefin may, for example, be a fluoro(alkyl vinyl ether) or a perfluoro(alkyl vinyl ether).

The monomer having no fluorine atom may, for example, be a monomer having a hydroxy group, or a vinyl-type monomer, that is, a compound having a carbon-carbon double bond.

The component other than the fluororesin is not particularly limited and may, for example, be a non-fluorinated resin or an additive.

The non-fluorinated resin is a polymer compound having no fluorine atom in its molecule and may, for example, be a polyester resin, an acrylic resin, an epoxy resin or a silicone resin.

The additive may, for example, be a known additive for a coating material and may, for example, be a film-forming aid, an inorganic coloring pigment, an organic coloring pigment, an extender pigment, a curing catalyst, a plasticizer, a preservative, a mildewproofing agent, an antifoaming agent, a leveling agent, a pigment dispersant, an anti-settling agent, an anti-sagging agent, a delustering agent, an ultraviolet absorber, a light stabilizer, a hydrophilic treatment agent, a water/oil repellent, an antistatic agent or an antioxidant.

The method for evaluating the weather resistance of the present invention is effective for evaluation of a fluorinated coating film containing at least a fluororesin, particularly effective for evaluation of a fluorinated coating film having a high content of the fluororesin and being more excellent in the weather resistance. Specifically, it is effective for evaluation of a fluorinated coating film containing a fluororesin in an amount of at least 5 mass% in 100 mass% of the fluorinated coating film, preferred for evaluation of a fluorinated coating film containing a fluororesin in an amount of at least 20 mass%, particularly preferred for evaluation of a fluorinated coating film containing a fluororesin in an amount of at least 40 mass%. Usually, it is preferred for evaluation of a fluorinated coating film containing a fluororesin in an amount of at most 70 mass%.

In a case where the fluorinated coating film contains a fluororesin and a non-fluororesin, the content of the fluororesin is preferably at least 30 mass%, more preferably at least 40 mass%, particularly preferably at least 50 mass% based on the total amount (100 mass%) of the fluororesin and the non-fluororesin. Further, the content of the fluororesin is preferably at most 80 mass%, more preferably 70 mass%.

Further, the fluorinated coating film may be one formed by a coating film in any form. As the form of the coating film, for example, a powder coating material, a solvent-based coating material or an aqueous coating material may be mentioned.

In the above-described method for evaluating the weather resistance of a fluorinated coating film, in the deterioration step (I), in order to wet the test piece, an aqueous hydrogen peroxide solution, not water, is used. Therefore, the weather resistance of a fluorinated coating film, which is superior in weather resistance to a usual coating film and which cannot be accurately evaluated by a conventional evaluation method e.g. as defined in JIS K5600-7-7 (2008) using water, can be evaluated accurately in a short time.

The results of evaluation of the weather resistance by the method for evaluating the weather resistance well correlate with the results of evaluation by an exposure test conducted outdoor in Naha, Okinawa for 3 years, as disclosed in the after-described Examples. Accordingly, the method is very effective as a method for evaluating the weather resistance of a fluorinated coating film in a hot and humid climate zone, and is particularly suitable for evaluation of a fluorinated coating film provided on an article (substrate) used outdoors. Such an article may, for example, be an exterior member (aluminum composite panel, aluminum panel for curtain wall, aluminum frame for curtain wall, aluminum window frame).

### <Method for producing fluorinated coating film>

In a case where the method for evaluating the weather resistance of the present invention is employed in production of a fluorinated coating film, a fluorinated coating film with high weather resistance can be selected based on the evaluation results. That is, the present invention provides a method for producing a fluorinated coating film, which comprises forming a fluorinated coating film on a substrate, evaluating the weather resistance of the fluorinated coating film by the above method for evaluating the weather resistance, and selecting a fluorinated coating film which has achieved an optional standard of weather resistance by the evaluation. According to the production method, it is possible to maintain a fluorinated coating film with high weather resistance and to suppress the dispersion of the weather resistance among the fluorinated coating films.

As the method for evaluating the weather resistance, the above method for evaluating the weather resistance may be applied in the same manner. As the optional standard of weather resistance, characteristics of the fluorinated coating film can be properly set, and the glossiness retention or the color difference is preferred with a view to selecting the fluorinated coating film in the evaluation step (II).

The glossiness retention may be properly set depending upon the conditions in the deterioration step (I) and is preferably at least 40%, more preferably at least 50%, further preferably at least 60%, most preferably at least 70%.

The color difference is preferably at most 5.0, more preferably at most 4.0, further preferably at most 3.0, most preferably at most 2.0.

### EXAMPLES

Now, the present invention will be described in further detail with reference to Examples. However, it should be understood that the present invention is by no means restricted to such specific Examples.

### <Test method, measurement method and evaluation method>

### [Accelerated weathering test (β)]

An exposure test was carried out under the following test conditions in accordance with JIS K5600-7-7 (method 1) using a xenon weather meter (manufactured by Suga Test Instruments Co., Ltd.).

### (Test conditions)

Relative humidity: 50%RH,
Black panel temperature: 63°C,
Irradiance of xenon arc radiation: 80 W/m² (300 to 400 nm),
Spraying and drying of water: a cycle of a drying time for 102 minutes and a spraying time for 18 minutes.

Specifically, drying (102 minutes) is a step of not spraying water with lamp irradiation, and spraying (18 minutes) is a step of spraying water with lamp irradiation. This cycle for 2 hours (102 minutes + 18 minutes) was repeatedly carried out until the total time reached 5,000 hours.

### (1) Glossiness retention

The proportion of the 60° glossiness after the test for 5,000 hours based on the 60° glossiness of the surface of the fluorinated coating film immediately before the test being 100% was calculated as the glossiness retention (unit: %).

### (2) Color difference (ΔE)

The colorimetry of the surface of the fluorinated coating film immediately before the test and the colorimetry of the surface of the fluorinated coating film after the test for 5,000 hours were measured by a colorimeter (manufactured by NIPPON DENSHOKU INDUSTRIES CO., LTD., SA4000). Measurement was conducted in accordance with JIS K5600-4-5: 1999. And, the color difference (ΔE) as between before and after the test was calculated in accordance with JIS K5600-4-6: 1999.

### [Accelerated weathering test (α)]

The accelerated weathering test (α) is a test in accordance with the evaluation method of the present invention.

In accordance with JIS K5600-7-7 (method 1), under the following test conditions, an exposure test was conducted by carrying out xenon arc radiation using a xenon weather meter. Here, instead of water, a 1 mass% aqueous hydrogen peroxide solution was sprayed on a fluorinated coating film (powder coating material) of a test piece to wet the film.

### (Test conditions)

Relative humidity: 70%RH,
Black panel temperature: 50°C,
Irradiance of xenon arc radiation: 80 W/m² (300 to 400 nm),
Spraying and drying of 1 mass% aqueous hydrogen peroxide solution: a cycle of spraying for 3 minutes and drying for 2 minutes.

### (1) Glossiness retention

The proportion of the 60° glossiness of the fluorinated coating film after exposed to xenon arc radiation for 40 hours based on the 60° glossiness of the fluorinated coating film immediately before xenon arc radiation being 100% is taken as the glossiness retention (unit: %). The glossiness retention was measured and calculated in accordance with JIS K5600-4-7: 1999 (ISO 2813:1994). Specifically, it was obtained in accordance with the above-described procedure (1a) to (9a) (provided that measurement of the color difference was omitted in (8a)).

### (2) Color difference (ΔE)

The colorimetry of the surface of the fluorinated coating film immediately before xenon arc radiation and the colorimetry of the surface of the fluorinated coating film after exposed to xenon arc radiation for 40 hours were measured by a colorimeter (manufactured by NIPPON DENSHOKU INDUSTRIES CO., LTD., SA4000). Measurement was conducted in accordance with JIS K5600-4-5: 1999. And, the color difference (ΔE) as between before and after the radiation was calculated in accordance with JIS K5600-4-6: 1999. Specifically, it was obtained in accordance with the above-described procedure (1 a) to (9a) (provided that measurement of the glossiness was omitted in (8a)).

### [Outdoor exposure test]

### (1) Glossiness retention

A test piece was installed outdoor in Naha, Okinawa, and the 60° specular glossiness of the surface of a fluorinated coating film immediately before the installation and the 60° specular glossiness of the surface of the fluorinated coating film 3 years later were measured in accordance with JIS K5600-4-7: 1999 (ISO2813: 1994) using a glossimeter (manufactured by NIPPON DENSHOKU INDUSTRIES CO., LTD., PG-1M). The proportion of the glossiness 3 years later based on the glossiness immediately before the installation being 100% was calculated as the glossiness retention (unit: %).

### (2) Color difference (ΔE)

A test piece was installed outdoor in Naha, Okinawa, and the colorimetry of the surface of the fluorinated coating film immediately before the installation, and the colorimetry of the surface of the fluorinated coating film 3 years later, were measured in accordance with JIS K5600-4-5:1999 using a colorimeter (manufactured by NIPPON DENSHOKU INDUSTRIES CO., LTD., SA4000), and further, the color difference (ΔE) as between before and after the test was calculated in accordance with JIS K5600-4-6: 1999.

### (1) Example of evaluation of fluorinated coating film prepared by using powder coating material

Resins, pigments, etc. used for production of a powder coating material are as follows.

### [Fluororesin (B)]

Fluororesin (B-1): a hydroxy group-containing fluorinated polymer (manufactured by Asahi Glass Company, Limited, LUMIFLON (tradename) LF710F, hydroxy value: 51.3 mgKOH/g, glass transition temperature: 55°C, number average molecular weight: 10,000).
Fluororesin (B-2): PVDF (manufactured by SHENZHOU NEWMATERIAL, PVDF DS203, mass average molecular weight: 270,000, number average molecular weight: 160,000).

### [Titanium oxide pigment (A)]

Titanium oxide pigment (A-1): Ti-Pure R960 (tradename, manufactured by Du Pont, titanium oxide content: 89 mass%, covering metal: silica, alumina).
Titanium oxide pigment (A-2): Tipaque CR97 (tradename, manufactured by Ishihara Sangyo Kaisha, Ltd., titanium oxide content: 93 mass%, covering metal: alumina, zirconia).
Titanium oxide pigment (A-3): TR-81 (tradename, manufactured by HUNTSMAN, titanium oxide content: 93 mass%, covering metal: alumina, zirconia).
Titanium oxide pigment (A-4): D918 (tradename, manufactured by SAKAI CHEMICAL INDUSTRY CO., LTD., titanium oxide content: 85 mass%, covering metal: silica, alumina, zirconia).

### [Curing agent (C)]

Curing agent (C-1): blocked isocyanate curing agent (manufactured by EVONIK INDUSTRIES, VESTAGON (tradename) B1530).

### [Non-fluororesin (D)]

Polyester resin (D2): CRYLCOAT 4890-0 (tradename, manufactured by DAICEL-ALLNEX LTD.)

### [Curing catalyst (F)]

Curing catalyst (F-1): xylene solution of dibutyltin dilaurate (10,000-fold diluted).

### [Other additives (H)]

Degassing agent: benzoin.
Surface conditioner: leveling agent for powder coating material (manufactured by BYK Additives & Instruments, BYK (tradename)-360P).

### [Ex. 1, 2 and 4 to 6]

Components in parts by mass as identified in Table 1 were mixed for about 10 to 30 minutes by using a high speed mixer (manufactured by Yusaki Co., Ltd.) to obtain a powdered mixture. Using a twin screw extruder (16 mm extruder, manufactured by Thermoprism Ltd.), the mixture was subjected to melt-kneading at a barrel set temperature of 120°C to obtain pellets made of a powder. Then, the obtained pellets were pulverized at room temperature using a pulverizer (rotor speed mill P14, manufactured by FRITSCH), followed by classification by a 150 mesh sieve, to obtain a powder coating material having an average particle size (50% average volume particle size distribution) of about 40 µm.

The obtained powder coating material was applied to one surface of an aluminum plate (75 mm x 150 mm x 1 mm in thickness) subjected to chromate treatment, by electrostatic coating by an electrostatic coating machine (manufactured by Onoda Cement Co., Ltd., GX3600C), followed by holding for 20 minutes in a 200°C atmosphere. The coated product was left to cool to room temperature to obtain an aluminum plate having a powder coating film with a thickness of 55 to 65 µm attached. Using the obtained aluminum plate with a powder coating material as a test piece, an outdoor exposure test and accelerated weathering tests (α) and (β) were conducted. The results of evaluation of the glossiness retention and the color difference are as shown in Table 1.

### [Ex. 3]

### (Production of acrylic resin (D1))

Into a 4-necked flask having an internal capacity of 1 L equipped with a condenser and a thermometer, 200 mL of deionized water, 2 g of a reactive emulsifier (manufactured by Sanyo Chemical Industries, Ltd., ELEMINOL (tradename) JS-2, succinate derivative) and 2 g of polyoxyethylene nonylphenyl ether (ethylene oxide 10 mole added) were charged. When the temperature reached 80°C in a warm bath under a nitrogen stream, 20 mL of a 2 mass% aqueous solution of ammonium persulfate was added. Then, a mixture of 140.2 g of methyl methacrylate, 80.0 g of ethyl methacrylate and 0.2 g of n-lauryl mercaptan as a chain transfer agent, was dropwise added over a period of 1 hour. Immediately thereafter, 2 mL of a 2 mass% aqueous solution of ammonium persulfate was added to initiate the reaction. After 3 hours, the temperature in the flask was raised to 85°C and held for one hour, followed by filtration through a 300 mesh wire gauge to obtain a blue-white aqueous dispersion. The obtained aqueous dispersion was frozen and coagulated at -25°C, followed by dehydration and washing, and vacuum drying at 80°C, to obtain 209.2 g of white powdery acrylic resin (D1). Acrylic resin (D1) had a glass transition temperature of 56.6°C, a mass average molecular weight of 92,000, and a number average molecular weight of 43,000.

Then, a powder coating material in Ex. 3 was obtained using components in parts by mass as identified in Table 1 except for the above acrylic resin (D1). The powder coating material was obtained in the same manner as in Ex. 1 except that the barrel set temperature was 190°C when pellets were obtained.

The obtained powder coating material was applied on one surface of an aluminum plate subjected to chromate treatment, by electrostatic coating by an electrostatic coating machine (manufactured by Onoda Cement Co., Ltd., GX3600C), followed by holding for 20 minutes in a 250°C atmosphere. Then, the coated product was left to cool to room temperature to obtain an aluminum plate having a powder coating film with a thickness of 55 to 65 µm attached. Using the obtained aluminum plate with a powder coating film as a test piece, an outdoor exposure test and accelerated weathering tests (α) and (β) were conducted. The results of evaluation of the glossiness retention and the color difference are as shown in Table 1.

**TABLE 1**

| Ex. | | 1 | 2 | 3 | 4 | 5 | 6 |
|---|---|---|---|---|---|---|---|
| Fluororesin (B) | (B-1) | 52.0 | 52.0 | - | 16.7 | - | - |
| | (B-2) | - | - | 45.5 | - | - | - |
| Curing agent (C) | | 13.0 | 13.0 | - | 9.5 | 9.0 | 9.0 |
| Acrylic resin (D1) | | - | - | 19.3 | - | - | - |
| Polyester resin (D2) | | - | - | - | 38.9 | 57.0 | 57.0 |
| Titanium oxide pigment (A) | (A-1) | - | - | 35.0 | 35.0 | 35.0 | - |
| | (A-2) | 35.0 | - | - | - | - | - |
| | (A-3) | - | 35.0 | - | - | - | 35.0 |
| Other additives | Curing catalyst | 0.005 | 0.005 | - | 0.005 | 0.005 | 0.005 |
| | Degassing agent | 0.4 | 0.4 | 0.4 | 0.4 | 0.4 | 0.4 |
| | Surface conditioner | 1.0 | 1.0 | 1.0 | 1.0 | 1.0 | 1.0 |
| Glossiness retention (%) | Accelerated weathering test (β) | 90 | 91 | 91 | 91 | 35.5 | 28.6 |
| | Accelerated weathering test (α) | 40 | 38 | 85 | 92 | 15.2 | 12.1 |
| | Exposure test | 39.2 | 36.9 | 85.1 | 88.1 | 38.2 | 29.4 |
| Color difference (ΔE) | Accelerated weathering test (β) | 2.7 | 2.6 | 2.5 | 1.9 | 8.3 | 10.2 |
| | Accelerated weathering test (α) | 5.3 | 6.2 | 1.9 | 1.8 | 9.2 | 11.3 |
| | Exposure test | 5.9 | 7.1 | 2.4 | 2.0 | 7.9 | 8.4 |

As shown in Table 1, in Ex. 1 and 2, significant deterioration was confirmed by the results in the outdoor exposure test (glossiness retention and color difference) in Okinawa, whereas a high glossiness retention was obtained in the accelerated weathering test (β) but results close to those in the outdoor exposure test in Okinawa were obtained in the accelerated weathering test (α). Therefore, the accelerated weathering test and the evaluation method in the present invention were found to be effective as a method for evaluating a fluorinated coating film.

In Ex. 5 and 6, in which a coating film made of a non-fluororesin (D) was used, deterioration excessively proceeded in the accelerated weathering test (α), and accordingly the results in the test (α) departed from the results in the outdoor exposure test in Okinawa rather than the accelerated weathering test (β).

### (2) Example of evaluation of fluorinated coating film prepared by using solvent-based coating film

As a fluororesin (B), fluororesin (B-3) which is a hydroxy group-containing fluororesin solution (manufactured by Asahi Glass Company, Limited, LUMIFLON (tradename) LF200, hydroxy value: 52.5 mgKOH/g, glass transition temperature: 35°C, number average molecular weight: 20,000, xylene solution with a solid content of 60 mass%) was used.

### [Ex. 7]

To 83 g of fluororesin (B-3), 200 g of titanium oxide pigment (A-4), 43 g of xylene and 43 g of butyl acetate were added, and 369 g of glass beads having a diameter of 1 mm were added, followed by stirring by a paint shaker for 2 hours. After stirring, the glass beads were removed by filtration to obtain a pigment composition.

To 100 g of the pigment composition, 150 g of a xylene solution (non-volatile content of 60 mass%) of fluororesin (B-3), 18.5 g of a HDI nurate type polyisocyanate resin (manufactured by Nippon Polyurethane Industry Co., Ltd., tradename "CORONATE HX") as a curing agent and dibutyltin dilaurate (4 to 10-fold diluted with xylene to 3 g) as a curing catalyst were further added, followed by mixing to obtain a coating material composition (solvent-based coating material).

The obtained coating material composition was applied to an aluminum plate subjected to chromate treatment by a film applicator so that the dry film thickness would be 40 µm and dried at a temperature of 23°C under a humidity of 50%RH for two weeks to prepare a test piece.

With respect to the obtained test piece, the glossiness retention (%) was evaluated and as a result, it was 93.0% in the accelerated weathering test (β), 85.0% in the accelerated weathering test (α) and 86.3% in the outdoor exposure test.

### [Ex. 8]

To 16.7 g of fluororesin (B-3), 40.0 g of titanium oxide pigment (A-4), 20.0 g of xylene and 23.2 g of butyl acetate were added, and 100.0 g of glass beads having a diameter of 1 mm were added, followed by stirring by a paint shaker for 2 hours. After stirring, the glass beads were removed by filtration to obtain a pigment composition.

Then, to 31.4 g of the pigment composition, 44.0 g of fluororesin (B-3), 10.2 g of a blocked isocyanate resin (manufactured by Sumitomo Bayer Urethane Co., Ltd., tradename "SUMIDUR BL3175") as a curing agent, 12.6 g of butyl acetate and dibutyltin dilaurate (4 to 10-fold diluted with xylene to 1.8 g) as a curing catalyst were further added, followed by mixing to obtain a coating material composition (solvent-based coating material).

The obtained coating material composition was applied to an aluminum plate subjected to chromate treatment by a film applicator so that the dry film thickness would be 40 µm, and cured by drying at a temperature of 160°C for 20 minutes to prepare a test piece.

With respect to the obtained test piece, the glossiness retention (%) was evaluated and as a result, it was 92.0% in the accelerated weathering test (β), 82.0% in the accelerated weathering test (α) and 84.2% in the outdoor exposure test.

### [Ex. 9]

To 16.7 g of fluororesin (B-3), 40.0 g of titanium oxide pigment (A-4), 20.0 g of xylene and 23.2 g of butyl acetate were added, and 100.0 g of glass beads having a diameter of 1 mm were added, followed by stirring by a paint shaker for 2 hours. After stirring, the glass beads were removed by filtration to obtain a pigment composition.

Then, to 31.4 g of the pigment composition, 44.0 g of fluororesin (B-3), 8.1 g of a methylated melamine resin (manufactured by Mitsui Cytec Ltd., tradename "CYMEL 303") as a curing agent, 12.2 g of butyl alcohol, 0.4 g of butyl acetate and 0.8 g of a p-toluenesulfonic acid solution neutralized with an amine compound as a curing catalyst (manufactured by Mitsui Cytec Ltd., tradename "CYMEL 303") were further added, followed by mixing to obtain a coating material composition (solvent-based coating material).

The obtained coating material composition was applied to an aluminum plate subjected to chromate treatment by a film applicator so that the dry film thickness would be 40 µm, and dried and cured at a temperature of 200°C for 10 minutes to prepare a test piece.

With respect to the obtained test piece, the glossiness retention (%) was evaluated and as a result, it was 90.0% in the accelerated weathering test (β), 80.0% in the accelerated weathering test (α) and 75.1% in the outdoor exposure test.

### [Ex. 10]

To 100 g of fluororesin (B-3), 10.7 g of a HDI nurate type polyisocyanate resin (manufactured by Nippon Polyurethane Industry Co., Ltd., tradename "CORONATE HX"), 100 g of xylene and dibutyltin dilaurate (4 to 10-fold diluted with xylene to 3 g) as a curing catalyst (D) were added, followed by mixing to prepare a coating material composition (solvent-based coating material).

The obtained coating material composition was applied to an aluminum plate subjected to chromate treatment by a film applicator so that the dry film thickness would be 40 µm, and dried and cured at room temperature for one week to prepare a test piece.

With respect to the obtained test piece, the glossiness retention (%) was evaluated and as a result, it was 99.0% in the accelerated weathering test (β), 85.0% in the accelerated weathering test (α) and 90.1% in the outdoor exposure test.

### (3) Example of evaluation of fluorinated coating film prepared by using aqueous coating material

As a fluororesin (B) in an aqueous coating material, the following resins were used.
Fluororesin (B-4): A hydroxy group-containing fluororesin aqueous dispersion (manufactured by Asahi Glass Company, Limited, LUMIFLON (tradename) FD1000, hydroxy value: 85.0 mgKOH/g, carboxy group: 15.0 mgKOH/g, number average molecular weight: 7,000, aqueous dispersion with a solid content of 40 mass%, average particle size: 80 nm).
Fluororesin (B-5): a PVDF-type fluororesin aqueous dispersion (manufactured by Arkema, Kynar Aquatec (tradename) FMA-12, aqueous dispersion with a solid content of 50 mass%).
Fluororesin (B-5): a PVDF-type fluororesin aqueous dispersion (manufactured by Arkema, Kynar Aquatec (tradename) FMA-12, aqueous dispersion with a solid content of 50 mass%).
Fluororesin (B-6): (manufactured by Asahi Glass Company, Limited, LUMIFLON (tradename) FE4300, hydroxy value: 10 mgKOH/g, aqueous dispersion with a solid content of 50 mass%).

### [Ex. 11]

### Preparation of pigment composition

210 parts by mass of titanium oxide pigment (A1-3), 21 parts by mass of a pigment dispersing agent (manufactured by BYK Additives & Instruments, Disperbyk (tradename) 190, copolymer having affinity for pigment, acid value: 10 mgKOH/g), 4.5 parts by mass of a defoaming agent (manufactured by Cognis, DEHYDRAN (tradename) 1620), 64.5 parts by mass of deionized water and 300 parts by mass of glass beads were mixed, followed by dispersion by a dispersing machine at 25°C for 2 hours, and the glass beads were removed by filtration to prepare a pigment composition.

Then, to 55 g of the above pigment composition, 193 g of fluororesin (B-4), 1.3 g of surface conditioner (manufactured by BYK Additives & Instruments, BYK (tradename)-348), 0.5 g of a thickener (manufactured by Akzo Nobel, BERMOCOLL (tradename) 2150) and 25 g of a water-dispersible isocyanate curing agent (manufactured by Sumika Bayer Urethane Co., Ltd., BAYHYDUR (tradename) 3100) were added, followed by mixing at 25°C for 10 minutes to prepare a coating material composition (aqueous coating material).

The obtained coating material composition (aqueous coating material) was applied to one side of an aluminum plate subjected to chromate treatment by a film applicator so that the dry film thickness would be 40 µm and dried at a temperature of 23°C under a humidity of 50%RH for two weeks to prepare a test piece.

With respect to the obtained test piece, the glossiness retention (%) was evaluated and as a result, it was 91.0% in the accelerated weathering test (β), 81.0% in the accelerated weathering test (α) and 71.5% in the outdoor exposure test.

### [Ex. 12]

A coating material composition (aqueous coating material) was prepared and a test piece was prepared in the same manner as in Ex. 14 except that fluororesin (B-5) was used instead of fluororesin (B-4) in Ex. 11, and 15 g of 2,2,4-trimethyl-1,3-pentadiol mono(2-methylpropanate) as a film-forming aid was used instead of the surface conditioner and the water-dispersible isocyanate curing agent.

With respect to the obtained test piece, the glossiness retention (%) was evaluated and as a result, it was 83.0% in the accelerated weathering test (β), 65.0% in the accelerated weathering test (α) and 61.5% in the outdoor exposure test.

### [Ex. 13]

193 g of fluororesin (B-6), 1.3 g of a surface conditioner (manufactured by BYK Additives & Instruments, BYK (tradename)-348) and 0.5 g of a thickener (manufactured by Akzo Nobel, BERMOCOLL (tradename) 2150) were mixed at 25°C for 10 minutes to prepare a coating material composition (aqueous coating material).

The obtained coating material composition (aqueous coating material) was applied to an aluminum plate subjected to chromate treatment by a film applicator so that the dry film thickness would be 40 µm, and dried at a temperature of 23°C under a humidity of 50%RH for two weeks to prepare a test piece.

With respect to the obtained test piece, the glossiness retention (%) was evaluated and as a result, it was 89.0% in the accelerated weathering test (β), 41.0% in the accelerated weathering test (α) and 59.0% in the outdoor exposure test.

As mentioned above, even with respect to fluorinated coating films prepared by using a solvent-based coating material and an aqueous coating material, the glossiness retention in the accelerated weathering test (α) in accordance with the evaluation method of the present invention was close to the glossiness retention in the outdoor exposure test in Okinawa, in the same manner as the fluorinated coating film prepared by using a powder coating material. That is, it was found that the method for evaluating the weather resistance of the present invention is effective as a method for evaluating a fluorinated coating film.

### INDUSTRIAL APPLICABILITY

The method for evaluating the weather resistance of a fluorinated coating film of the present invention is very effective as a method for evaluating the weather resistance of a fluorinated coating film, and is particularly suitable for evaluation of a fluorinated coating film to be provided on an article (substrate) used outdoors. Such an article may, for example, be an exterior member (such as aluminum composite panel, aluminum panel for curtain wall, aluminum frame for curtain wall and aluminum window frame).

The entire disclosures of Japanese Patent Application No. 2014-118759 filed on June 9, 2014, Japanese Patent Application No. 2014-177540 filed on September 1, 2014 and Japanese Patent Application No. 2014-177541 filed on September 1, 2014 including specifications, claims, drawings and summaries are incorporated herein by reference in their entireties.

## Claims

1. A method for evaluating the weather resistance of a fluorinated coating film, which comprises a fluorinated coating film deterioration step (I) comprising a step (ia) of exposing the fluorinated coating film to xenon arc radiation and a step (ib) of attaching an aqueous hydrogen peroxide solution to the fluorinated coating film under xenon arc radiation to wet the fluorinated coating film, and an evaluation step (II) of comparing characteristics of the fluorinated coating film as between before and after the deterioration step (I) to evaluate the weather resistance of the fluorinated coating film.

2. The method for evaluating the weather resistance according to Claim 1, wherein the irradiance of the xenon arc radiation in the step (ia) is from 50 to 400 W/m² within a wavelength range of from 300 to 400 nm.

3. The method for evaluating the weather resistance according to Claim 1 or 2, wherein in the step (ib), the concentration of the aqueous hydrogen peroxide solution is from 0.05 to 10.0 mass%.

4. The method for evaluating the weather resistance according to any one of Claims 1 to 3, wherein the step (ib) is carried out more than once.

5. The method for evaluating the weather resistance according to any one of Claims 1 to 4, which further comprises a drying step (ic) after the step (ib) is carried out and before the evaluation step (II) is carried out.

6. The method for evaluating the weather resistance according to any one of Claims 1 to 5, wherein the weather resistance is evaluated by at least one selected from the group consisting of glossiness, color difference, outer appearance of the coating film by observation with a scanning electron microscope, mapping of F atoms and titanium atoms by energy dispersive X-ray spectroscopy, and peak variation of fluororesin by infrared analysis.

7. The method for evaluating the weather resistance according to any one of Claims 1 to 6, wherein the total time of the xenon arc radiation is from 1 to 10,000 hours.

8. The method for evaluating the weather resistance according to any one of Claims 1 to 7, wherein the fluorinated coating film contains at least one fluororesin in an amount of at least 5 mass% in the fluorinated coating film (100 mass%), and has a film thickness of from 10 to 100 µm.

9. The method for evaluating the weather resistance according to any one of Claims 1 to 8, wherein the fluorinated coating film is formed by using a powder coating material, a solvent-based coating material or an aqueous coating material.

10. A method for producing a fluorinated coating film, which comprises forming a fluorinated coating film on a substrate, evaluating the weather resistance of the fluorinated coating film by the method for evaluating the weather resistance as defined in any one of Claims 1 to 9, and selecting a fluorinated coating film which has achieved an optional standard of weather resistance by the evaluation.
